# FASCICULE DE BREVET EUROPEEN

(11) **EP 0 486 394 B1**
(45) Date de publication et mention de la délivrance du brevet: **02.11.1994**
(21) Numéro de dépôt: 91403058.0
(22) Date de dépôt: 14.11.1991
(51) Int. Cl.: A61K 7/48, A61K 7/035, A61K 7/00

(54) **Compositions cosmétiques sous forme de poudres coulées comprenant des microphères creuses, et leur préparation**
Mikrohohlkugeln enthaltende gegossene feste kosmetische Puder-Zubereitung und Verfahren zur Herstellung derselben
Moulded powder cake cosmetic composition, containing hollow microspheres and process for producing the same

(30) Priorité: 15.11.1990 FR 9014224
(43) Date de publication de la demande: 20.05.1992
(73) Titulaire: L'OREAL, 75008 Paris (FR)
(72) Inventeur: Gagnebien, Didier, F-92320 Chatillon (FR); Defossez, Béatrice, F-75020 Paris (FR); Lecomte, Sophie, F-75013 Paris (FR)
(74) Mandataire: Tonnellier, Jean-Claude

(56) Documents cités:
- EP-A- 0 211 298
- EP-A- 0 254 612
- WORLD PATENTS INDEX LATEST Derwent Publications Ltd.,London, GB; AN 85-272732(44) &
- JP-A-60 184 004 (POLA KASEI KOGYO K.K.) 19 Septembre 1985

## Description

La présente invention a pour objet une composition cosmétique solide sous forme de poudre coulée comprenant une charge principale particulaire constituée par des microsphères creuses comportant une ou plusieurs cavités ouvertes ou fermées.

L'invention concerne également un procédé d'obtention de telles poudres coulées.

On sait que certains produits de maquillage sous la forme de produits cosmétiques solides poudreux, tels que des fards à paupières ou des fards à joues, sont généralement présentés sous la forme de poudres dites coulées. On appelle "poudre coulée" le produit obtenu en mélangeant une phase particulaire solide et un agent liant (généralement une phase grasse) dans un solvant, pour obtenir une pâte fluide, en répartissant ladite pâte fluide par coulage dans des conteneurs appropriés (coupelles), puis en évaporant le solvant. Les avantages des poudres coulées sont d'une part la possibilité d'introduire dans la composition des ingrédients variés grâce au choix d'un solvant adapté, et d'autre part le libre choix de la forme et du matériau de la coupelle, ce qui permet des présentations multiples du produit fini.

Dans la préparation des poudres coulées, on observe plusieurs sortes d'inconvénients : un phénomène de retrait qui fait que la coupelle n'est pas complètement remplie (ce qui indispose le consommateur) ; un aspect irrégulier de la surface du produit (aspect ridé, fissuration et formation de cratères au moment de l'évaporation) ; et formation d'un film de l'agent liant en surface.

Diverses solutions ont été proposées pour remédier à ces inconvénients.

Dans la demande de brevet EP 0038645, la coupelle, dont la partie supérieure ouverte est munie d'un couvercle amovible, est remplie, en position inversée, par le fond qui est constitué par un support en forme de grille. Après évaporation du solvant, on obture le fond avec une plaque de fond appropriée. De cette façon, l'aspect irrégulier de la surface à travers laquelle s'est produite l'évaporation du solvant, de même que le phénomène de retrait, ne sont pas visibles par le consommateur. Ce procédé présente l'inconvénient de nécessiter une coupelle spéciale avec le support de fond en forme de grille.

Le brevet américain 4,804,538 prévoit l'utilisation d'une coupelle dont le fond comporte des ouvertures pour un remplissage avec la pâte fluide par le fond, en position non inversée, à l'aide de buses d'injection alimentées sous pression, tandis que le solvant est évaporé par la partie supérieure de la coupelle reliée à une pompe à vide. Ce procédé nécessite une installation complexe et coûteuse.

Dans la demande de brevet EP 0165137, on a proposé l'utilisation comme liant d'une bentonite traitée avec un tensioactif cationique. Ce liant n'étant pas soluble dans le solvant, la formation d'un film superficiel de l'agent liant est évitée. Mais le problème du retrait n'est pas résolu, et le toucher du produit obtenu n'est pas satisfaisant.

Le brevet DE 3327001 décrit un procédé de préparation de poudres coulées dans lequel les diverses opérations de mélange des ingrédients avec un excès de solvant, de coulée et d'évaporation du solvant sont effectuées à chaud, ce qui augmente les coûts de production. Selon ce brevet, l'état de la surface de la poudre coulée est amélioré. Toutefois, le phénomène de retrait n'est pas évité.

Il a maintenant été découvert que l'addition, comme constituant de la charge particulaire, d'une faible proportion pondérale de microsphères creuses présentant une ou plusieurs cavités ouvertes ou fermées, permet de préparer, avec une diminution importante du phénomène de retrait, des poudres coulées ayant un état de surface et un délitage satisfaisants.

Il a été également mis en évidence que l'utilisation de ces nouveaux constituants de la charge particulaire permet une préparation à une température modérée et avec un choix de solvants divers, ce qui présente bien entendu des avantages sur le plan économique.

L'invention est donc relative à une composition cosmétique solide sous forme de poudre coulée constituée d'une phase particulaire solide et éventuellement d'un agent liant et d'additifs usuels, caractérisée par le fait que la phase particulaire comprend des microsphères creuses présentant une ou plusieurs cavités, ouvertes ou fermées.

Il a été constaté que par la mise en oeuvre de l'invention on peut obtenir une poudre solide ayant un état de surface et un délitage satisfaisants, et un taux de retrait très faible. On notera ici que, comme cela est bien connu des spécialistes, les poudres coulées ont l'aspect et les propriétés d'un produit solide cohérent, ayant notamment, contrairement aux poudres libres (s'écoulant librement), une surface définie. Comme il s'agit de produits solides cohérents, leur utilisation nécessite le prélèvement d'une certaine quantité de poudre par délitage, à l'aide d'un applicateur approprié.

Les microsphères utilisés sont ici des particules de forme sensiblement sphérique ayant généralement un diamètre inférieur à 70µm (de préférence inférieur à 40 µm).

Les microsphères sont réalisées en tout matériau organique ou inorganique approprié, compatible avec une utilisation sur la peau, c'est-à-dire non irritant et non toxique.

Les microsphères en matériau organique polymère possédant une seule cavité fermée contenant un gaz tel qu'un hydrocarbure (par exemple l'isobutane) peuvent être préparées selon les procédés connus, par exemple ceux décrits dans le brevet US 3,615,972 et dans la demande de brevet EP 0056219.

Les microsphères organiques sont réalisées par exemple en polymères ou copolymères dérivés d'acides, d'amides ou d'esters (monomères) à insaturation éthylénique, en polymères urée-formaldéhyde, en polymères ou copolymères de chlorure de vinylidène, etc.

On peut citer à titre d'exemple les microsphères faites de polymères ou copolymères d'acrylate ou de méthacrylate de méthyle, ou encore de copolymères chlorure de vinylidène/acrylonitrile.

Parmi les copolymères chlorure de vinylidène-acrylonitrile, on citera notamment ceux qui contiennent, en poids, de 20 à 60 % de motifs dérivés de chlorure de vinylidène, de 20 à 60 % en poids de motifs dérivés d'acrylonitrile, et de 0 à 40 % en poids d'autres motifs tels que des motifs dérivés d'un monomère acrylique ou styrénique.

On peut encore utiliser des polymères ou copolymères acryliques reticulés, par exemple des polymères dont les groupements carboxyliques sont partiellement estérifiés par des diols servant d'agents réticulants.

Ces matériaux peuvent servir de base à la préparation de microsphères microporeuses.

Lorsque les microsphères creuses utilisées selon l'invention sont des microsphères microporeuses, leur portée correspond par exemple à une surface spécifique d'au moins 0,5 m²/g, et en particulier d'au moins 1 m²/g. Il n'y a pas de limite supérieure (autre que celle résultant de la possibilité de fabriquer en pratique des microsphères à porosité très élevée) pour la surface spécifique. La surface spécifique peut atteindre par exemple 1000 m²/g ou même bien davantage, par exemple jusqu'à quelques centaines de milliers de m²/g.

Particulièrement préférées sont notamment les microsphères microporeuses vendues par la Société DOW CORNING sous la dénomination POLYTRAP Q5-6603, ou celles vendues par la Société SEPPIC sous la dénomination MICROPEARL M ou MICROPEARL M 100, ainsi que les microsphères creuses à cavité fermée vendues sous la dénomination EXPANCEL par la Société KEMA NORD PLAST.

Parmi les microsphères dérivées de matériaux inorganiques, on peut citer à titre d'exemple des microparticules de silice à porosités ouvertes ou, de préférence, creuses, telles que par exemple celles vendues sous la dénomination SILICA BEADS S700 par la Société MIYOSHI KASEI INC.

De préférence, on utilise des microsphères ayant une densité (plus exactement une masse volumique) inférieure à 0,7 g/cm³, par exemple comprise entre 0,01 et 0,7 g/cm³. La masse volumique des poudres dépend de leur degré de tassement. Les masses volumiques données ici correspondant aux masses volumiques mesurées selon la norme allemande DIN53194, après dix tassements.

La proportion pondérale des microsphères creuses dans les poudres coulées varie généralement de 0,2 à 15 % par rapport au poids total de la composition avec le solvant (pâte fluide).

En ce qui concerne les proportions de microsphères creuses dans la composition finale séchée, elles peuvent varier généralement de 0,1 à 50 % et de préférence de 0,5 à 15 % par rapport au poids total de la composition après évaporation du solvant.

La composition sous forme de poudre coulée contenant des microsphères ainsi définies peut contenir en outre des pigments et/ou d'autres charges complémentaires. Il s'agit bien entendu de produits particulaires non creux (autrement dit, qui ne comportent sensiblement pas de cavité ouverte ou fermée) et dont les dimensions ne dépassent pas 200 µm environ.

Les pigments et/ou charges complémentaires sont ceux habituellement utilisés dans les compositions cosmétiques sous forme de poudres solides. Les pigments sort choisis notamment parmi les pigments minéraux, les pigments organiques ou leurs mélanges.

Parmi les pigments minéraux, on peut citer, à titre d'exemples :
- le dioxyde de titane (rutile ou anatase) éventuellement traité en surface et codifié dans le Color Index sous la référence CI 77891 ;
- les oxydes de fer noir, jaune, rouge et brun, codifiés sous les références CI 77499, 77492, 77491 ;
- le violet de manganèse (CI 77742) ;
- le bleu outremer (CI 77007) ;
- l'oxyde de chrome hydraté (CI 77289) ; et
- le bleu ferrique (CI 77510).

Parmi les pigments organiques, on peut citer, en particulier, les pigments :
D & C red n° 19 (CI 45170) ;
D & C red n° 9 (CI 15585) ;
D & C red n° 21 (CI 45380) ;
D & C orange n° 4 (CI 15510) ;
D & C orange n° 5 (CI 45370) ;
D & C red n° 27 (CI 45410) ;
D & C red n° 13 (CI 15630) ;
D & C red n° 7 (CI 15850-1) ;
D & C red n° 6 (CI 15850-2) ;
D & C yellow n° 5 (CI 19140) ;
D & C red n° 36 (CI 12085) ;
D & C orange n°10 (CI 45425) ;
D & C yellow n° 6 (CI 15985) ;
D & C red n° 30 (CI 73360) ;
D & C red n° 3 (CI 45430) ;
le noir de carbone (CI 77266) ; et
les laques à base de carmin de cochenille (CI 75470).

On peut utiliser également des pigments nacrés qui peuvent être choisis notamment parmi :
- les pigments nacrés blancs, tels que le mica recouvert d'oxyde de titane, l'oxychlorure de bismuth ; et
- les pigments nacrés colorés, tels que le mica titane avec des oxydes de fer, le mica titane avec du bleu ferrique, ou de l'oxyde de chrome, le mica titane avec un pigment organique du type précité, ainsi que ceux à base d'oxychlorure de bismuth.

Les pigments peuvent représenter jusqu'à 50 % du poids total de la pâte fluide.

Les charges complémentaires sont choisies notamment parmi :
- le talc, qui est un silicate de magnésium hydraté, utilisé sous forme de particules généralement de dimensions inférieures à 40 µm ;le talc possède des propriétés absorbantes de l'humidité et est utilisé surtout en raison de son toucher onctueux ;
- les micas, qui sont des aluminosilicates de compositions variées, qui se présentent sous la forme d'écailles ayant des dimensions de 2 à 200 µm, de préférence de 5 à 70 µm et une épaisseur 0,1 à 5 µm, de préférence de 0,2 à 3 µm. Les micas peuvent être d'origine naturelle (par exemple muscovite, margarite, roscoelithe, lépidolithe, biotite) ou d'origine synthétique. Les micas sont généralement transparents et permettent de conférer à la peau un aspect satiné ;
- l'amidon, en particulier l'amidon de riz ;
- le kaolin, qui est un silicate d'aluminium hydraté, qui se présente sous la forme de particules de forme isotrope ayant des dimensions généralement inférieure à 30 µm, et qui possède de bonnes propriétés d'absorption des corps gras ;
- les oxydes de zinc et de titane, généralement utilisés sous la forme de particules ayant des dimensions ne dépassant pas quelques micromètres (ou même inférieures à 1 µm dans le cas de l'oxyde de titane) ; ces oxydes ont un toucher onctueux, ont un bon pouvoir couvrant et ont une opacité importante ;
- le carbonate de calcium précipité qui, sous forme de particules de dimensions inférieures à 10 µm, a un toucher onctueux et permet d'obtenir un aspect mat ;
- le carbonate ou l'hydrocarbonate de magnésium, qui possèdent notamment des propriétés de fixation des parfums ;
- des savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium, etc. Ces savons, présents généralement sous la forme de particules ayant des dimensions inférieures de 10 µm ont un toucher onctueux et facilitent l'adhérence de la poudre sur la peau ;
- Les poudres de polymères synthétiques, tels que le polyéthylène, les polyesters (par exemple isophtalate ou téréphtalate de polyéthylène), les polyamides, sous la forme de particules ayant des dimensions inférieures à 50 µm, qui possèdent des propriétés absorbantes, et permettent de conférer à la peau un aspect velouté.

Ces charges complémentaires peuvent représenter jusqu'à 65 % du poids total de la pâte fluide.

Le véhicule liquide non aqueux (appelé généralement "solvant") utilisé pour mettre en suspension les différents constituants peut être choisi par exemple dans le groupe constitué par : les alcools (tels que l'éthanol ou l'isopropanol) ; les alcanes à chaîne droite ou ramifiée tels que l'hexane, le pentane, l'heptane ou les isoparaffines ; les alcanes cycliques (tels que le cyclohexane) ; les silicones volatiles (par exemple les cyclométhicones) ; les solvants chlorés (tels que le dichlorométhane ou le dichloroéthane) ; et leurs mélanges. Ce solvant représente 30 à 70 % du poids de la pâte fluide, et de préférence 45 à 65 %.

De préférence, comme mentionné ci-dessus, la suspension contient une phase grasse qui peut agir en tant que liant. Cette phase grasse est constituée d'au moins un corps gras, liquide ou solide à température ambiante et/ou d'au moins un polymère synthétique oléosoluble dont l'utilisation dans les cosmétiques est connue.

Parmi les corps gras liquides à température ambiante, on peut citer les huiles minérales, animales, végétales ou synthétiques, ou encore les huiles de silicone. Il s'agit par exemple de l'huile de vaseline, de lanoline liquide, l'huile d'arara, les huiles de sésame, de macadamia ou de jojoba, les triglycérides de synthèse.

Parmi les polymères synthétiques oléosolubles, on peut citer les copolymères polyvinylpyrrolidone/hexadécène ou PVP/eicosène tels que les produits vendus par GAF Corp. sous les dénominations GANEX V-216 et GANEX V-220.

La phase grasse représente généralement de 0 à 20 % du poids total de la pâte fluide.

Divers autres additifs peuvent être introduits dans la pâte fluide, tels que : des filtres solaires ; des agents adoucissants ; des agents hydratants (sorbitol, glycérine) ; des agents cicatrisants ; des anti-radicaux libres ; des vitamines ; des parfums ; etc.

Enfin, des agents de consistance hydrosolubles telles que des gommes naturelles ou synthétiques, des dérivés de cellulose ou des polymères acryliques, peuvent être ajoutés au cours de la préparation de la pâte fluide.

On prépare la composition cosmétique de l'invention par un procédé dans lequel on disperse dans un véhicule liquide non aqueux une phase particulaire et éventuellement un agent liant et des additifs usuels, pour obtenir une pâte fluide homogène, on répartit ladite pâte fluide dans des conteneurs appropriés, et on évapore ledit véhicule liquide, caractérisé par le fait que ladite phase particulaire comprend des microsphères creuses comportant une ou plusieurs cavités (ouvertes ou fermées).

Le procédé de l'invention consiste donc, dans un premier temps, à introduire les différents constituants dans le véhicule liquide. Pour mettre en suspension les différents constituants du produit cosmétique dans ce solvant, il est possible de mélanger préalablement les constituants poudreux entre eux et les constituants de la phase grasse entre eux. Toutefois, il est également possible d'introduire les différents constituants dans le solvant dans un ordre quelconque. On homogénéise ensuite le mélange, généralement à température ambiante. Ce mélange est alors coulé directement dans des coupelles, de préférence à température ambiante. Enfin, on évapore le solvant. Pour cela, on peut placer les coupelles à l'étuve, de préférence à une température ne dépassant pas 40°C environ, pendant un temps suffisant qui peut varier de 6 à 60 heures.

Les exemples donnés ci-après illustrent l'invention

### EXEMPLE 1 : FARD A PAUPIERES

### Phase A

| | |
|---|---|
| Talc | 9,7 |
| Oxyde de chrome | 1,8 |
| Bleu outremer | 0,7 |
| Stéarate de zinc | 0,7 |
| Mica | 5,3 |
| Amidon | 1,7 |
| Micatitane | 10,5 |
| EXPANCEL 551 DE (Kemanord Plast) | 1,4 |

### Phase B

| | |
|---|---|
| Copolymère polyvinylpyrrolidone hexadécène | 0,2 |
| Huile de jojoba | 0,5 |
| Myristate d'isopropyle | 0,7 |
| Lanoline | 0,4 |
| Huile d'amande douce | 1,2 |

### Conservateurs

| | |
|---|---|
| Butylhydroxytoluène (BHT) | 0,015 |
| Butylhydroxyanisole (BHA) | 0,015 |
| Parahydroxybenzoate de propyle | 0,1 |

### Solvant

| | |
|---|---|
| Cyclométhicone | 65 |

La poudre Expancel 551DE est constituée de microsphères creuses (cavité unique) en copolymère chlorure de vinylidène-acrylonitrile (masse volumique 0,02 g/cm³).
Pour réaliser ce fard à paupières, on procède de la façon suivante :
Dans un premier temps on mélange entre eux les constituants de la phase B. On introduit par ailleurs dans un mélangeur les constituants de la phase A, puis le solvant, et on homogénéise le tout.
Puis la phase B, dans laquelle ont été ajoutés les conservateurs (BHT, BHA et parahydroxybenzoate de propyle), est introduite à son tour dans le mélangeur et on mélange à nouveau jusqu'à obtention d'une pâte homogène. La pâte est coulée dans des coupelles métalliques à température ambiante. Ces coupelles sont ensuite placées à l'étuve à 40°C pendant 55 heures. A l'issue de cette période, on obtient un fard à paupières bleu-vert dont la surface est lisse et homogène et qui est particulièrement doux à l'application.

### EXEMPLE 2 : FARD A PAUPIERES

### PHASE A

| | |
|---|---|
| Talc | 13,4 |
| Oxydes de fer | 5,8 |
| Micatitane | 13,7 |
| EXPANCEL 551 DE (Kemanord Plast) | 2,0 |

### PHASE B

| | |
|---|---|
| Huile de vaseline | 2,0 |
| Lanoline | 2,0 |

### Conservateur :

| | |
|---|---|
| Parahydroxybenzoate de propyle | 0,1 |

### Solvant

| | |
|---|---|
| Cyclohexane | 61 |

Pour réaliser ce fard à paupières, on procède de la façon suivante : on mélange la phase A et la phase B, avec le solvant, dans le mélangeur, et on agite le tout jusqu'à obtention d'une pâte homogène. La poudre est coulée dans des coupelles métalliques à température ambiante. Les coupelles sont ensuite placées à l'étuve à 40°C pendant 20 heures.

On a préparé de façon analogue, un fard à paupières selon l'invention en remplaçant l'huile de vaseline et la lanoline par 4 g de stéaryldiméthicone.

### EXEMPLE 3 : FARD A JOUES

### PHASE A

| | |
|---|---|
| Talc | 20,8 |
| Dioxyde de titane | 3,5 |
| Micatitane coloré | 16 |
| DC Red 30 | 0,1 |
| POLYTRAP Q5-6603 (Dow Corning) | 1,5 |

### PHASE B

| | |
|---|---|
| Huile de vaseline | 2,5 |
| Huile de jojoba | 0,5 |

### Conservateur

| | |
|---|---|
| Parahydroxybenzoate de propyle | 0,1 |

### Solvant

| | |
|---|---|
| Ethanol | 55 |

La poudre POLYTRAP Q5-6603 est constituée de microsphères creuses en copolymère d'acrylate réticulé, comportant des cavités ouvertes (environ 13,6 % du volume total ; surface spécifique 9,6 m²/g environ).

Pour réaliser ce fard à joues, on procède comme dans l'exemple 1.

De façon analogue, on a préparé un fard à joues selon l'invention en remplaçant l'huile de jojoba par la même quantité de triglycéride caprylique/caprique.

### EXEMPLE 4 : POUDRE POUR LE VISAGE

### PHASE A

| | |
|---|---|
| Talc | 27,3 |
| Oxydes de fer | 4 |
| Amidon | 2 |
| Dioxyde de titane | 1,5 |
| MICROPEARL M100 (SEPPIC) | 3,5 |
| Mica | 9 |

### PHASE B

| | |
|---|---|
| Myristate d'isopropyle | 1,5 |
| Huile de vaseline | 0,6 |
| Sorbitol | 0,4 |

### Conservateurs

| | |
|---|---|
| Parahydroxybenzoate de propyle | 0,1 |
| Butylhydroxyanisole | 0,05 |
| Butylhydroxytoluène | 0,05 |

### Solvant

| | |
|---|---|
| Hydrocarbure isoparaffinique | 50 |

La poudre MICROPEARL M100 est constituée de microsphères de polyméthacrylate de méthyle comportant des cavités ouvertes (surface spécifique : 0,9-1 m²/g environ).

Pour préparer cette poudre pour le visage, on procède de la façon analogue à celle décrite dans l'exemple 1.

### EXEMPLE 5 : POUDRE POUR LE VISAGE

### PHASE A

| | |
|---|---|
| Talc | 29 |
| Oxychlorure de bismuth | 6 |
| Dioxyde de titane | 4 |
| Amidon | 6,5 |
| Oxydes de fer | 2 |
| POLYTRAP Q5-6603 | 3,5 |

### PHASE B

| | |
|---|---|
| Huile de jojoba | 1,5 |
| Lanoline | 0,5 |

### Solvant

| | |
|---|---|
| Isopropanol | 47 |

Pour préparer cette poudre pour le visage, on procède de façon analogue à celle décrite dans l'exemple 1.

### EXEMPLE 6 : POUDRE PARFUMEE POUR LE CORPS

### PHASE A

| | |
|---|---|
| Talc | 33,37 |
| Oxydes de fer | 0,05 |
| Stéarate de zinc | 1,5 |
| POLYTRAP Q5-6603 | 1 |

### PHASE B et conservateur

| | |
|---|---|
| Parahydroxybenzoate de propyle | 0,08 |
| Parfum | 2 |

### Solvant

| | |
|---|---|
| Dichlorométhane | 62 |

Pour préparer cette poudre parfumée pour le corps, on procède de façon analogue à celle décrite dans l'exemple 1.

### EXEMPLE 7 : FARD A PAUPIERES

### PHASE A

| | |
|---|---|
| Oxyde de chrome | 5,88 |
| Micatitane | 13,72 |
| Talc | 14,94 |
| EXPANCEL 551 DE | 1,18 |

### PHASE B et conservateur

| | |
|---|---|
| Huile de vaseline | 2,12 |
| Lanoline | 0,19 |
| Alcool oléique | 0,39 |
| Vaseline | 0,39 |
| Myristate d'isopropyle | 0,31 |
| Parahydroxybenzoate de propyle | 0,08 |

### Solvant

| | |
|---|---|
| Cyclohexane | 60,8 |

Pour préparer ce fard à paupières, on procède de façon analogue à celle décrite dans l'exemple 1.

### EXEMPLE 8 : FARD A PAUPIERES

### PHASE A

| | |
|---|---|
| Oxyde de chrome | 5,88 |
| Micatitane | 13,72 |
| Talc | 14,94 |
| POLYTRAP Q5-6603 | 1,18 |

### PHASE B et conservateur

| | |
|---|---|
| Huile de vaseline | 2,12 |
| Lanoline | 0,19 |
| Alcool oléique | 0,39 |
| Vaseline | 0,39 |
| Myristate d'isopropyle | 0,31 |
| Parahydroxybenzoate de propyle | 0,08 |

### Solvant

| | |
|---|---|
| Cyclohexane | 60,8 |

Pour préparer ce fard à paupières, on procède de façon analogue à celle décrite dans l'exemple 1.

### EXEMPLE 9 : FARD A PAUPIERES

### PHASE A

| | |
|---|---|
| Oxyde de chrome | 5,88 |
| Micatitane | 13,72 |
| Talc | 14,94 |
| MICROPEARL M-100 | 1,18 |

### PHASE B et conservateur

| | |
|---|---|
| Huile de vaseline | 2,12 |
| Lanoline | 0,19 |
| Alcool oléique | 0,39 |
| Vaseline | 0,39 |
| Myristate d'isopropyle | 0,31 |
| Parahydroxybenzoate de propyle | 0,08 |

### Solvant

| | |
|---|---|
| Cyclohexane | 60,8 |

Pour préparer ce fard à paupières, on procède de façon analogue à celle décrite dans l'exemple 1.

### EXEMPLE 10 : FARD A PAUPIERES

### PHASE A

| | |
|---|---|
| Oxyde de chrome | 5,88 |
| Micatitane | 13,72 |
| Talc | 14,94 |
| SILICA BEADS SB700 | 1,18 |

### PHASE B et conservateur

| | |
|---|---|
| Huile de vaseline | 2,12 |
| Lanoline | 0,19 |
| Alcool oléique | 0,39 |
| Vaseline | 0,39 |
| Myristate d'isopropyle | 0,31 |
| Parahydroxybenzoate de propyle | 0,08 |

### Solvant

| | |
|---|---|
| Cyclohexane | 60,8 |

La poudre SILICABEADS SB700 est une poudre de particules de silice poreuses présentant une cavité centrale (surface spécifique : environ 600-800m²/g).

Pour préparer ce fard à paupières, on procède de façon analogue à celle décrite dans l'exemple 1.

### EXEMPLES DE COMPARAISON

Les exemples 11 à 14 suivants sont des exemples de comparaison.

### EXEMPLE 11 : FARD A PAUPIERES

### PHASE A

| | |
|---|---|
| Oxyde de chrome | 5,88 |
| Micatitane | 13,72 |
| Talc | 14,94 |
| Polyamide * | 1,18 |

| | |
|---|---|
| *Poudre de polyamide vendue sous la dénomination commerciale RILSAN D50 NAT par ATOCHEM. | |

### PHASE B et conservateur

| | |
|---|---|
| Huile de vaseline | 2,12 |
| Lanoline | 0,19 |
| Alcool oléique | 0,39 |
| Vaseline | 0,39 |
| Myristate d'isopropyle | 0,31 |
| Parahydroxybenzoate de propyle | 0,08 |

### Solvant

| | |
|---|---|
| Cyclohexane | 60,8 |

Pour préparer ce fard à paupière, on procède de façon analogue à celle décrite dans l'exemple 1.

### EXEMPLE 12 : FARD A PAUPIERES

### PHASE A

| | |
|---|---|
| Oxyde de chrome | 5,88 |
| Micatitane | 13,72 |
| Talc | 14,94 |
| Polyéthylène * | 1,18 |

| | |
|---|---|
| *Poudre de polyéthylène vendue sous la dénomination commerciale COATHYLENE HA 1681 par PLAT-LABOR. | |

### PHASE B et conservateur

| | |
|---|---|
| Huile de vaseline | 2,12 |
| Lanoline | 0,19 |
| Alcool oléique | 0,39 |
| Vaseline | 0,39 |
| Myristate d'isopropyle | 0,31 |
| Parahydroxybenzoate de propyle | 0,08 |

### Solvant

| | |
|---|---|
| Cyclohexane | 60,8 |

Pour préparer ce fard à paupières, on procède de façon analogue à celle décrite dans l'exemple 1.

### EXEMPLE 13 : FARD A PAUPIERES

### PHASE A

| | |
|---|---|
| Oxyde de chrome | 5,88 |
| Micatitane | 13,72 |
| Talc | 14,94 |
| Polyvinylpyrrolidone * | 1,18 |

| | |
|---|---|
| *Poudre vendue sous la dénomination commerciale PVP K-30 par GAF. | |

### PHASE B et conservateur

| | |
|---|---|
| Huile de vaseline | 2,12 |
| Lanoline | 0,19 |
| Alcool oléique | 0,39 |
| Vaseline | 0,39 |
| Myristate d'isopropyle | 0,31 |
| Parahydroxybenzoate de propyle | 0,08 |

### Solvant

| | |
|---|---|
| Cyclohexane | 60,8 |

Pour préparer ce fard à paupières, on procède de façon analogue à celle décrite dans l'exemple 1.

### EXEMPLE 14 : FARD A PAUPIERES

### PHASE A

| | |
|---|---|
| Oxyde de chrome | 5,88 |
| Micatitane | 13,72 |
| Talc | 14,94 |
| Phosphate tricalcique pulvérulent | 1,18 |

### PHASE B et conservateur

| | |
|---|---|
| Huile de vaseline | 2,12 |
| Lanoline | 0,19 |
| Alcool oléique | 0,39 |
| Vaseline | 0,39 |
| Myristate d'isopropyle | 0,31 |
| Parahydroxybenzoate de propyle | 0,08 |

### Solvant

| | |
|---|---|
| Cyclohexane | 60,8 |

Pour préparer ce fard à paupières, on procède de façon analogue à celle décrite dans l'exemple 1.

### ESSAIS COMPARATIFS

Afin de mettre en évidence les qualités des produits cosmétiques obtenus selon l'invention, des essais comparatifs ont été effectués.

Ces essais consistent à comparer 4 compositions selon l'invention (exemples 7 à 10) avec 4 compositions dans lesquelles les microsphères étaient remplacées par des charges classiques (constituée de particules ne comportant pas de cavité ouverte ou fermée), à savoir une poudre de nylon, une poudre de polyéthylène, la polyvinylpyrrolidone et la phosphate tricalcique (exemples 11 à 14 respectivement).

Les essais ont été effectués pour étudier (1) le délitage et (2) le retrait dans la coupelle.

### 1)DELITAGE

Le délitage est étudié grâce à un appareil à déliter constitué d'un bras animé par un disque rotatif et équipé d'un applicateur à l'une de ses extrémités. Cet applicateur vient se poser sur la surface du produit coulé et séché où il effectue un nombre déterminé de rotations. L'appareil permet d'exercer une force constante sur le produit coulé. On mesure alors la perte en poids moyenne sur trois coupelles après 10 tours de délitage. Le délitage, caractérisé par la perte en poids (Δp) en g pour chacune des huit compositions testées, est donné dans le tableau ci-après.

### 2) RETRAIT DANS LA COUPELLE

Afin de déterminer la hauteur de remplissage de la coupelle après évaporation du solvant, on emploie le comparateur INTERAPID (Ludwig Metrologie 1515, Société ROCH) qui permet la mesure d'une épaisseur allant jusqu'à 10 mm avec une précision de 10⁻² mm.

Pour chacune des huit compositions, on a effectué une série de cinq mesures par coupelle, répétées sur six coupelles soit au total une moyenne sur trente mesures.

On établit ainsi la hauteur moyenne de remplissage de la coupelle. Cette hauteur, divisée par la hauteur de la coupelle utilisée (4 mm) donne le taux de remplissage (R, exprimé en %) de la coupelle après le séchage. Le taux de retrait est la différence entre 100 % et R. Les taux de remplissage pour chaque composition sont donnés dans le tableau ci-après.

| EXEMPLE | p (g) | R (%) |
|---|---|---|
| INVENTION | | |
| 7 | 4.10⁻³ | 99,7 |
| 8 | 6.10⁻³ | 89,7 |
| 9 | 10.10⁻³ | 88,3 |
| 10 | 10.10⁻³ | 89,0 |

| COMPARAISON | | |
|---|---|---|
| 11 | 22.10⁻³ | 50,5 |
| 12 | 20.10⁻³ | 57,2 |
| 13 | 21.10⁻³ | 51,5 |
| 14 | 23.10⁻³ | 50,5 |

Les résultats montrent que la perte en poids des compositions selon l'invention est réduite par rapport à celle des compositions de comparaison qui sont trop poudreuses.

Il est également remarquable que les compositions selon l'invention donnent un meilleur remplissage que les compositions comparatives. Le taux de retrait n'excède pas 10 % environ, alors qu'il est voisin de 50 % pour les compositions de comparaison.

## Revendications

1. Composition cosmétique solide caractérisée par le fait qu'elle se présente sous la forme d'une poudre coulée, et qu'elle comprend une charge particulaire contenant des microsphères creuses comportant une ou plusieurs cavités ouvertes ou fermées, la proportion pondérale desdites microsphères creuses représentant de 0,1 à 50 % du poids total de la composition.

2. Composition selon la revendication 1, caractérisée par le fait que les microsphères creuses ont une masse volumique inférieure à 0,7 g/cm³.

3. Composition selon la revendication 1 ou 2, caractérisée par le fait que les microsphères creuses ont un diamètre inférieur à 70 µm, et en particulier inférieur à 40 µm.

4. Composition selon l'une quelconque des revendications précédentes, caractérisée par le fait que lesdites microsphères sont des microsphères poreuses ayant une surface spécifique au moins égale à 0,5 m²/g, et en particulier au moins égale à 1 m²/g.

5. Composition selon l'une quelconque des revendications précédentes, caractérisée par le fait que les microsphères sont présentes à raison de 0,5 à 15 % par rapport au poids total de la composition.

6. Composition selon l'une quelconque des revendications précédentes, caractérisée par le fait que les microsphères creuses sont constituées d'un matériau polymère ou copolymère organique.

7. Composition selon l'une quelconque des revendications précédentes, caractérisé par le fait que lesdites microsphères creuses sont constituées en un matériau choisi parmi les polymères ou copolymères dérivés de monomères acides, amides et/ou esters à insaturation éthylénique, les polymères urée-formaldéhyde ou les polymères ou copolymères de chlorure de vinylidène.

8. Composition selon la revendication 7, caractérisée par le fait que ledit matériau est un copolymère contenant, en poids, de 20 à 60 % de motifs dérivés de chlorure de vinylidène, de 20 à 60 % en poids de motifs dérivés d'acrylonitrile, et de 0 à 40 % en poids d'autres motifs tels que des motifs dérivés d'un monomère acrylique ou styrénique.

9. Composition selon la revendication 7, caractérisée par le fait que ledit matériau est un polymère ou copolymère d'acrylate ou de méthacrylate de méthyle, ou est un polymère ou copolymère acrylique réticulé.

10. Composition selon l'une quelconque des revendications 1 à 5, caractérisée par le fait que les microsphères creuses sont constituées de silice.

11. Procédé de préparation d'une composition cosmétique solide telle que définie dans l'une quelconque des revendications précédentes, dans lequel on disperse dans un véhicule liquide non aqueux une phase particulaire et éventuellement un agent liant et des additifs usuels, pour obtenir une pâte fluide homogène, on répartit ladite pâte fluide dans des conteneurs appropriés, et on évapore ledit véhicule liquide, caractérisé par le fait que ladite phase particulaire comprend des microsphères creuses comportant une ou plusieurs cavités (ouvertes ou fermées).

12. Procédé selon la revendication 11, caractérisé par le fait que ledit véhicule liquide est choisi parmi les alcools, les alcanes à chaîne droite ou ramifiée, les alcanes cycliques, les silicones volatiles, les solvants chlorés et leur mélanges.

13. Procédé selon la revendication 12, caractérisé par le fait que le solvant est choisi parmi l'éthanol, l'isopropanol, le cyclohexane, l'hexane, le pentane, l'heptane, les isoparaffines, les cyclométhicones, le dichlorométhane et le dichloroéthane.

14. Utilisation, dans la préparation d'une composition cosmétique solide sous forme de poudre coulée, d'une charge particulaire constituée de microsphères creuses comportant une ou plusieurs cavités ouvertes ou fermées, ladite charge particulaire étant présente en une proportion pouvant aller de 0,1 à 50 % en poids par rapport au poids total de la composition.

15. Utilisation selon la revendication 14, caractérisée par le fait que ladite composition est telle que définie dans l'une quelconque des revendications 1 à 10.

16. Utilisation d'une charge particulaire constituée de microsphères creuses comportant une ou plusieurs cavités ouvertes ou fermées, dans la préparation d'une pâte fluide contenant une phase particulaire dispersée dans un véhicule liquide non aqueux, ladite pâte fluide étant destinée à être répartie dans des conteneurs appropriés de façon à obtenir, apres évaporation dudit véhicule liquide, une composition cosmétique solide sous la forme d'une poudre coulée ayant un état de surface et un délitage satisfaisants, avec un faible taux de retrait.

17. Utilisation selon la revendication 16, caractérisée par le fait que lesdites microsphères creuses sont telles que définies dans l'une quelconque des revendications 2 à 10.

18. Utilisation selon la revendication 16 ou 17, caractérisée par le fait que le véhicule liquide est tel que défini dans l'une quelconque des revendications 12 et 13.

## Claims

1. Solid cosmetic composition, characterized in that it takes the form of a cast powder, and in that it comprises a particulate filler containing hollow microspheres possessing one or more open or closed cavities, the weight proportion of the said hollow microspheres representing from 0.1 to 50% of the total weight of the composition.

2. Composition according to Claim 1, characterized in that the hollow microspheres have a bulk density of less than 0.7 g/cm³.

3. Composition according to Claim 1 or 2, characterized in that the hollow microspheres are less than 70 µm, and especially less than 40 µm, in diameter.

4. Composition according to any one of the preceding claims, characterized in that the said microspheres are porous microspheres having a specific surface area equal to at least 0.5 m²/g, and especially equal to at least 1 m²/g.

5. Composition according to any one of the preceding claims, characterized in that the microspheres are present in a proportion of 0.5 to 15% relative to the total weight of the composition.

6. Composition according to any one of the preceding claims, characterized in that the hollow microspheres consist of an organic polymeric or copolymeric material.

7. Composition according to any one of the preceding claims, characterized in that the said hollow microspheres consist of a material chosen from polymers or copolymers derived from ethylenically unsaturated acid, amide and/or ester monomers, urea-formaldehyde polymers or vinylidene chloride polymers or copolymers.

8. Composition according to Claim 7, characterized in that the said material is a copolymer containing, by weight, from 20 to 60% of units derived from vinylidene chloride, from 20 to 60% by weight of units derived from acrylonitrile and from 0 to 40% by weight of other units such as units derived from an acrylic or styrene monomer.

9. Composition according to Claim 7, characterized in that the said material is a methyl acrylate or methacrylate polymer or copolymer, or is a crosslinked acrylic polymer or copolymer.

10. Composition according to any one of Claims 1 to 5, characterized in that the hollow microspheres consist of silica.

11. Process for preparing a solid cosmetic composition as is defined in any one of the preceding claims, in which a particulate phase and, where appropriate, a binding agent and standard additives are dispersed in a non-aqueous liquid vehicle to obtain a homogeneous fluid paste, the said fluid paste is distributed in suitable containers and the said liquid vehicle is evaporated off, characterized in that the said particulate phase comprises hollow microspheres possessing one or more (open or closed) cavities.

12. Process according to Claim 11, characterized in that the said liquid vehicle is chosen from alcohols, alkanes having an unbranched or branched chain, cyclic alkanes, volatile silicones, chlorinated solvents, and mixtures thereof.

13. Process according to Claim 12, characterized in that the solvent is chosen from ethanol, isopropanol, cyclohexane, hexane, pentane, heptane, isoparaffins, cyclomethicones, dichloromethane and dichloroethane.

14. Use, in the preparation of a solid cosmetic composition in the form of a cast powder, of a particulate filler consisting of hollow microspheres possessing one or more open or closed cavities, the said particulate filler being present in a proportion which can range from 0.1 to 50% by weight relative to the total weight of the composition.

15. Use according to Claim 14, characterized in that the said composition is as defined in any one of Claims 1 to 10.

16. Use of a particulate filler consisting of hollow microspheres possessing one or more open or closed cavities, in the preparation of a fluid paste containing a particulate phase dispersed in a non-aqueous liquid vehicle, the said fluid paste being intended for distribution in suitable containers so as to obtain, after evaporation of the said liquid vehicle, a solid cosmetic composition in the form of a cast powder whose surface state and disintegration are satisfactory, with a low degree of shrinkage.

17. Use according to Claim 16, characterized in that the said hollow microspheres are as defined in any one of Claims 2 to 10.

18. Use according to Claim 16 or 17, characterized in that the liquid vehicle is as defined in either of Claims 12 and 13.

## Patentansprüche

1. Feste kosmetische Zubereitung, dadurch gekennzeichnet, daß sie in Form eines festen, gegossenen Puders vorliegt und daß sie einen Mikrohohlkugeln enthaltenden, teilchenförmigen Anteil umfaßt, wobei die Mikrohohlkugeln einen oder mehrere offene oder geschlossene Hohlräume aufweisen und der Gewichtsanteil der Mikrohohlkugeln 0,1 bis 50 Gew.- % des Gesamtgewichts der Zubereitung ausmacht.

2. Zubereitung gemäß Anspruch 1, dadurch gekennzeichnet, daß die Mikrohohlkugeln eine Dichte unterhalb von 0,7 g/cm³ aufweisen.

3. Zubereitung gemäß Anspruch 1 oder 2, dadurch gekennzeichnet, daß die Mikrohohlkugeln einen Durchmesser kleiner als 70 µm und insbesondere von kleiner als 40 µm haben.

4. Zubereitung gemäß einem der vorstehenden Ansprüche, dadurch gekennzeichnet, daß die Mikrohohlkugeln poröse Mikrokugeln mit einer spezifischen Oberflächen von mindestens gleich 0,5 m²/g und insbesondere von mindestens 1 m²/g sind.

5. Zubereitung gemäß einem der vorstehenden Ansprüche, dadurch gekennzeichnet, daß die Mikrokugeln in einem Verhältnis von 0,5 bis 15 %, bezogen auf das Gesamtgewicht der Zubereitung, vorliegen.

6. Zubereitung gemäß einem der vorstehenden Ansprüche, dadurch gekennzeichnet, daß die Mikrohohlkugeln aus einem polymeren oder copolymeren organischen Material bestehen.

7. Zubereitung gemäß einem der vorstehenden Ansprüche, dadurch gekennzeichnet, daß die Mikrohohlkugeln aus einem Material bestehen, welches aus Polymeren oder Copolymeren, die von Säure-, Amid- und/ oder Estermonomeren mit einer ungesättigten Bindung abgeleitet sind, Harnstoff-Formaldehyd-Polymeren oder Vinylchloridpolymeren oder -coplymeren ausgewählt ist.

8. Zubereitung gemäß Anspruch 7, dadurch gekennzeichnet, daß das besagte Material ein Polymer ist, welches zwischen 20 und 60 Gew.-% von Vinylchlorid abgeleitete Einheiten, zwischen 20 und 60 Gew.-% von Acrylnitril abgeleitete Einheiten und von 0 bis 40 Gew.-% weitere Einheiten enthält, wie z.B. solche, die von einem Acryl- oder Styrolmonomer abgeleitete Einheiten sind.

9. Zubereitung gemäß Anspruch 7, dadurch gekennzeichnet, daß das Material ein Acryl- oder Methylmethacrylatpolymer oder -copolymer oder auch ein netzartiges Acrylpolymer oder -copolymer ist.

10. Zubereitung gemäß einem der Ansprüche 1 bis 5, dadurch gekennzeichnet, daß die Mikrohohlkugeln aus Silika bestehen.

11. Verfahren zur Herstellung einer festen kosmetischen Zubereitung gemäß einem der vorstehenden Ansprüche, bei dem man in einem nichtwäßrigen flüssigen Träger eine teilchenförmige Phase sowie gegebenenfalls ein Bindemittel und übliche Zusätze zum Erhalt einer homogenen, fluiden Paste dispergiert, die fluide Paste in geeignete Behältnisse aufteilt und den flüssigen Träger abdampft, wobei das Verfahren dadurch gekennzeichnet ist, daß die teilchenförmige Phase einen oder mehrere (offene oder geschlossene) Hohlräume aufweisende Mikrohohlkugeln umfaßt.

12. Verfahren gemäß Anspruch 12, dadurch gekennzeichnet, daß der flüssige Träger aus Alkoholen, linearen oder verzweigten Alkanen, cyclischen Alkanen, flüchtigen Silikonen, chlorierten Lösungsmitteln sowie deren Gemischen ausgewählt ist.

13. Verfahren gemäß Anspruch 12, dadurch gekennzeichnet, daß das Lösungsmittel aus Ethanol, Isopropanol, Cyclohexan, Hexan, Pentan, Heptan, Isoparaffinen, Cyclomethikonen, Dichlormethan und Dichlorethan ausgewählt ist.

14. Verwendung eines teilchenförmigen Anteils in einer festen kosmetischen Zubereitung in Form eines gegossenen Puders, wobei der teilchenförmige Anteil aus einen oder mehrere Hohlräume tragenden Mikrohohlkugeln besteht und in einem Verhältnis von 0,1 bis 50 Gew.-% vorliegt, bezogen auf das Gesamtgewicht der Zubereitung.

15. Verwendung gemäß Anspruch 14, dadurch gekennzeichnet, daß die Zubereitung eine der gemäß einem der Ansprüche 1 bis 10 definierten Zubereitungen ist.

16. Verwendung eines teilchenförmigen Anteils, bestehend aus einen oder mehrere, offene oder geschlossene Hohlräume tragenden Mikrohohlkugeln, zur Herstellung einer fluiden Paste, welche einen teilchenförmigen Anteil dispergiert in einem flüssigen, nichtwäßrigen Träger enthält, wobei die fluide Paste durch Aufteilung auf geeignete Behältnisse in der erwünschten Form und anschließendes Abdampfen des flüssigen Trägers zum Erhalt einer festen kosmetischen Zubereitung in Form eines gegossenen Puders mit zufriedenstellender Oberflächenbeschaffenheit und Schichtung sowie mit geringer Schrumpfneigung bestimmt ist.

17. Verwendung gemäß Anspruch 16, dadurch gekennzeichnet, daß als Mikrohohlkugeln solche in Betracht kommen, die gemäß einem der Ansprüche 2 bis 10 definiert sind.

18. Verwendung gemäß einem der Ansprüche 16 oder 17, dadurch gekennzeichnet, daß als flüssiger Träger ein solcher in Betracht kommt, wie er gemäß einem der Ansprüche 12 und 13 definiert ist.
